# EUROPEAN PATENT SPECIFICATION

(11) **EP 3 248 859 B1**
(45) Date of publication and mention of the grant of the patent: **12.12.2018**
(21) Application number: 16196634.6
(22) Date of filing: 31.10.2016
(51) Int. Cl.: B62K 11/00, B60R 25/25

(54) **ANTI-THEFT METHOD AND DEVICE FOR A BALANCE VEHICLE**
DIEBSTAHLSICHERUNGSVERFAHREN UND -VORRICHTUNG FÜR EIN GLEICHGEWICHTSFAHRZEUG
DISPOSITIF ET PROCÉDÉ ANTIVOL POUR UN VÉHICULE À AUTO-ÉQUILIBRAGE

(30) Priority: 23.05.2016 CN 201610346479
(43) Date of publication of application: 29.11.2017
(73) Proprietor: Beijing Xiaomi Mobile Software Co., Ltd., Beijing 100085 (CN)
(72) Inventor: LIN, Shangquan, Beijing, Beijing 100085 (CN); YONG, Xing, Beijing, Beijing 100085 (CN); HA, Xiaolin, Beijing, Beijing 100085 (CN); ZHANG, Xiaotong, Beijing, Beijing 100085 (CN)
(74) Representative: dompatent von Kreisler Selting Werner - Partnerschaft von Patent- und Rechtsanwälten mbB

(56) References cited:
- US-A1- 2010 033 315
- US-A1- 2010 152 976
- US-A1- 2011 130 925
- US-A1- 2014 266 623
- US-A1- 2016 001 782

## Description

### FIELD

The present disclosure generally relates to the technical field of a balance vehicle, and more particularly to an anti-theft method and device for a balance vehicle.

### BACKGROUND

The balance vehicle is a tool for short distance riding instead of walk which is rapidly developed recently. The balance vehicle may alleviate the traffic pressure in the city to a certain extent, and may also be a popular element of street fashion. The balance vehicle is small and light, these portable features may be its advantage, meanwhile the balance vehicle may be stolen easily, namely the balance vehicle may be stolen by a thief easily when the owner lacks attention. Therefore, how to prevent the balance vehicle for being stolen has become a technical problem to be solved.
US 2010/0033315 A1 describes a balance vehicle with an alarm in order to detect unauthorized use of the vehicle.

### SUMMARY

Embodiments of the present disclosure provide an anti-theft method and device for a balance vehicle. The technical solutions are as follows.

According to a first aspect of the invention, an anti-theft method for a balance vehicle is provided, comprising: obtaining first physiological characteristics information of a current user on the balance vehicle; obtaining predetermined second physiological characteristics information; outputting prompt information to prompt that the current user is different from a predetermined user in response to a determination that the second physiological characteristics information excludes or is different from the first physiological characteristics information. It is preferred that a threshold is defined for this difference: If the difference between the first physiological characteristics information and the second physiological characteristics information is bigger than this threshold, it is assumed that the current user is different from a predetermined user. In other words, it is analyzed, if the value of the first physiological characteristics is in an acceptable error range compared to the value of the second physiological characteristics. When it is in this error range, it can be decided that the first physiological characteristics information is the same (or similar) to the second physiological characteristics information. The same threshold can be used in the inventive device.

In one embodiment, the first physiological characteristics information further comprises weight.

In one embodiment, the first physiological characteristics information comprises the weight; the obtaining first physiological characteristics information of a current user on the balance vehicle comprises: obtaining the weight of the current user on the balance vehicle via a gravity sensor installed on the balance vehicle.

According to the first aspect of the invention, the first physiological characteristics information comprises the area occupied by a foot; the obtaining first physiological characteristics information of a current user on the balance vehicle comprises: obtaining the area occupied by a foot of the current user on the balance vehicle via a pressure sensor installed on the balance vehicle.

In one embodiment, the outputting prompt information comprises at least one of: controlling the balance vehicle to output the prompt information; controlling a terminal associated with the balance vehicle to output the prompt information; wherein, the prompt information is outputted by at least one of displaying content and playing voice.

In one embodiment, the method further comprising: obtaining an update instruction inputted with respect to the second physiological characteristics information; updating the second physiological characteristics information according to the update instruction; wherein, the update instruction comprises at least one of an adding instruction, a deleting instruction and a modifying instruction.

In one embodiment, when the update instruction comprises at least one of the adding instruction and the modifying instruction, the updating the second physiological characteristics information according to the update instruction comprises: obtaining inputted physiological characteristics information according to the update instruction; updating the second physiological characteristics information according to the inputted physiological characteristics information.

In one embodiment, the obtaining inputted physiological characteristics information comprises: obtaining the physiological characteristics information inputted via a terminal; or obtaining the physiological characteristics information of a user on the balance vehicle.

In one embodiment, the method further comprising: disabling the current user to use the balance vehicle in response to a determination that the second physiological characteristics information excludes the first physiological characteristics information; enabling the current user to use the balance vehicle in response to a determination that the second physiological characteristics information includes the first physiological characteristics information.

In one embodiment, the method further comprising: obtaining geographical location information where the balance vehicle is currently located in response to a determination that the second physiological characteristics information excludes the first physiological characteristics information; outputting the geographical location information to the terminal associated with the balance vehicle.

In one embodiment, the method further comprising: obtaining geographical location information where the balance vehicle is currently located in response to a determination that the second physiological characteristics information excludes the first physiological characteristics information; outputting the geographical location information to the terminal associated with the balance vehicle.

According to a second aspect of the invention, an anti-theft device for a balance vehicle is provided, comprising: a first obtaining module configured to obtain first physiological characteristics information of a current user on the balance vehicle; a second obtaining module configured to obtain predetermined second physiological characteristics information; a prompt module configured to output prompt information to prompt that the current user is different from a predetermined user in response to a determination that the second physiological characteristics information obtained by the second obtaining module excludes or is different from the first physiological characteristics information obtained by the first obtaining module.

In one embodiment, the first physiological characteristics information further comprises weight.

In one embodiment, the first physiological characteristics information comprises the weight; the first obtaining module comprises: a first obtaining submodule configured to obtain the weight of the current user on the balance vehicle via a gravity sensor installed on the balance vehicle.

According to the second aspect of the invention, the first physiological characteristics information comprises the area occupied by a foot; the first obtaining module comprises: a second obtaining submodule configured to obtain the area occupied by a foot of the current user on the balance vehicle via a pressure sensor installed on the balance vehicle.

In one embodiment, the prompt module comprises at least one of: a first output submodule configured to control the balance vehicle to output the prompt information; a second output submodule configured to control a terminal associated with the balance vehicle to output the prompt information; wherein, the prompt information is outputted by at least one of displaying content and playing voice.

In one embodiment, the device further comprising: a third obtaining module configured to obtain an update instruction inputted with respect to the second physiological characteristics information; an updating module configured to update the second physiological characteristics information according to the update instruction obtained by the third obtaining module; wherein, the update instruction comprises at least one of an adding instruction, a deleting instruction and a modifying instruction.

In one embodiment, when the update instruction comprises at least one of the adding instruction and the modifying instruction, the updating module comprises: an information obtaining submodule configured to obtain inputted physiological characteristics information according to the update instruction; an updating submodule configured to update the second physiological characteristics information according to the inputted physiological characteristics information obtained by the information obtaining submodule.

In one embodiment, the information obtaining submodule is configured to: obtain the physiological characteristics information inputted via a terminal; or obtain the physiological characteristics information of a user on the balance vehicle.

In one embodiment, the device further comprising: a first processing module configured to disable the current user to use the balance vehicle in response to a determination that the second physiological characteristics information excludes the first physiological characteristics information; a second processing module configured to enable the current user to use the balance vehicle in response to a determination that the second physiological characteristics information includes the first physiological characteristics information.

In a third aspect, an anti-theft device for a balance vehicle is provided, comprising: a processor; a memory for storing processor-executable instructions; wherein the processor is configured to: obtain first physiological characteristics information of a current user on the balance vehicle; obtain predetermined second physiological characteristics information; output prompt information to prompt that the current user is different form a predetermined user in response to a determination that the second physiological characteristics information excludes or is different from the first physiological characteristics information.

Embodiments of the present disclosure may provide at least some of the following beneficial effects:

The above technical solution may obtain first physiological characteristics information of a current user on the balance vehicle (such as weight and area occupied by a foot of the current user), obtain stored predetermined second physiological characteristics information (such as physiological characteristics information of the owner of the balance vehicle), and output prompt information to prompt the user that the current user is different form a predetermined user when the second physiological characteristics information excludes or is different from the first physiological characteristics information (namely the physiological characteristics information of the current user is not the predetermined physiological characteristics information), such that the balance vehicle may be prevented from being stolen and the property loss of the user may be avoided.

It is to be understood that the forgoing general description and the following detailed description are illustrative only, and are not intended to limit the present disclosure.

### BRIEF DESCRIPTION OF THE DRAWINGS

The accompanying drawings, which are incorporated in and constitute a part of this specification, illustrate embodiments consistent with the present disclosure and, together with the description, serve to explain the principles of the present disclosure.
Fig. 1 is a flow diagram illustrating an anti-theft method for a balance vehicle according to an exemplary embodiment.
Fig. 2 is a flow diagram illustrating another anti-theft method for a balance vehicle according to an exemplary embodiment.
Fig. 3 is a flow diagram illustrating another anti-theft method for a balance vehicle according to an exemplary embodiment.
Fig. 4 is a flow diagram illustrating another anti-theft method for a balance vehicle according to an exemplary embodiment.
Fig. 5 is a flow diagram illustrating step S402 of another anti-theft method for a balance vehicle according to an exemplary embodiment.
Fig. 6 is a flow diagram illustrating another anti-theft method for a balance vehicle according to an exemplary embodiment.
Fig. 7 is a flow diagram illustrating another anti-theft method for a balance vehicle according to an exemplary embodiment.
Fig. 8 is a block diagram illustrating an anti-theft device for a balance vehicle according to an exemplary embodiment.
Fig. 9 is a block diagram illustrating a first obtaining module of an anti-theft device for a balance vehicle according to an exemplary embodiment.
Fig. 10 is a block diagram illustrating a first obtaining module of another anti-theft device for a balance vehicle according to an exemplary embodiment.
Fig. 11 is a block diagram illustrating a prompt module of another anti-theft device for a balance vehicle according to an exemplary embodiment.
Fig. 12 is a block diagram illustrating another anti-theft device for a balance vehicle according to an exemplary embodiment.
Fig. 13 is a block diagram illustrating an updating module of another anti-theft device for a balance vehicle according to an exemplary embodiment.
Fig. 14 is a block diagram illustrating another anti-theft device for a balance vehicle according to an exemplary embodiment.
Fig. 15 is a block diagram illustrating an anti-theft device for a balance vehicle according to an exemplary embodiment.

### DETAILED DESCRIPTION

Reference will now be made in detail to example embodiments, examples of which are illustrated in the accompanying drawings. The following description refers to the accompanying drawings in which same numbers in different drawings represent same or similar elements unless otherwise described. The implementations set forth in the following description of example embodiments do not represent all implementations consistent with the present disclosure. Instead, they are merely examples of devices and methods consistent with aspects related to the present disclosure as recited in the appended claims.

The embodiments of the present disclosure provide an anti-theft method for a balance vehicle, the method may be applied into a balance vehicle and other terminal devices for controlling the balance vehicle or having a data connection to the balance vehicle, wherein, the terminal devices may be mobile phones, computers, digital broadcasting terminals, message transceivers, game consoles, tablet devices, medical devices, fitness devices, personal digital assistants and the like.

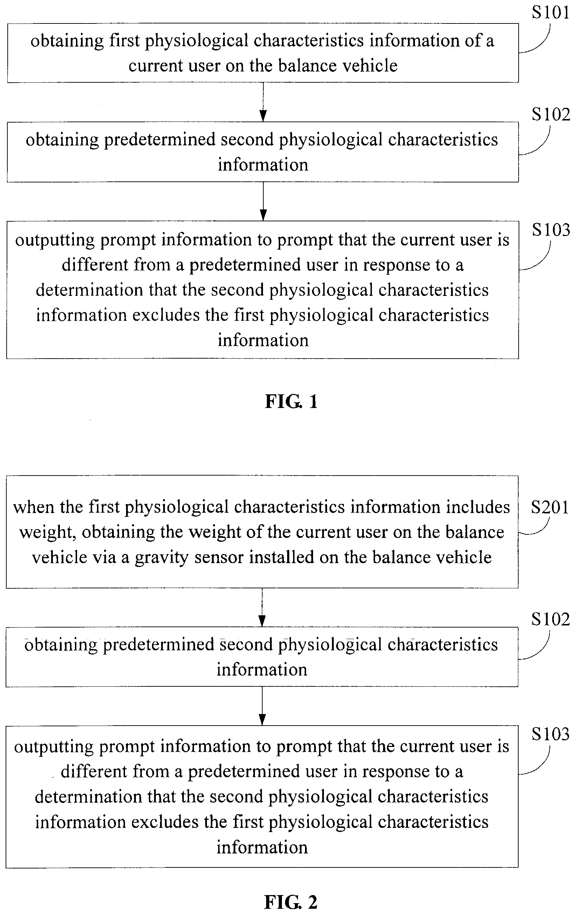

Fig. 1 is a flow diagram illustrating an anti-theft method for a balance vehicle according to an exemplary embodiment. As shown in Fig. 1, the method may include steps S101 to S103.

In step S101, first physiological characteristics information of a current user on the balance vehicle may be obtained.

In one embodiment, the first physiological characteristics information may include at least one of weight and area occupied by a foot.

In step S102, predetermined second physiological characteristics information may be obtained.

The predetermined second physiological characteristics information may be predetermined physiological characteristics information of a owner of a balance vehicle or physiological characteristics information of a user associated with the owner, such as physiological characteristics information of a friend or family of the owner. Such that, the owner may prestore the physiological characteristics information of the user enabled to use the balance vehicle so as to ensure that the user may use the balance vehicle normally.

In step S103, prompt information may be outputted to prompt that the current user is different from a predetermined user in response to a determination that the second physiological characteristics information excludes the first physiological characteristics information.

In the embodiment, the above technical solution may obtain first physiological characteristics information of a current user on the balance vehicle (such as weight and area occupied by a foot of the current user), obtain stored predetermined second physiological characteristics information (such as physiological characteristics information of the owner of the balance vehicle), and output prompt information to prompt the user that the current user is different form a predetermined user when the second physiological characteristics information excludes the first physiological characteristics information (namely the physiological characteristics information of the current user is not the predetermined physiological characteristics information), such that the balance vehicle may be prevented from being stolen and the property loss of the user may be avoided.

The second physiological characteristics information may be one or more, and may belong to one user or multiple users. When determining whether the second physiological characteristics information includes the first physiological characteristics information or not, it is expected to determine whether the first physiological characteristics information is the same as or similar to the second physiological characteristics information. Whether it is similar means whether the value of the first physiological characteristics is in an acceptable error range of the value of the second physiological characteristics, when it is in the error range, it may be determined that the second physiological characteristics information includes the first physiological characteristics information.

Fig. 2 is a flow diagram illustrating another anti-theft method for a balance vehicle according to an exemplary embodiment. As shown in Fig. 2, when the first physiological characteristics information includes weight, the above step S101 may include step S201.

In step S201, the weight of the current user on the balance vehicle may be obtained via a gravity sensor installed on the balance vehicle.

In the embodiment, the weight of the current user on the balance vehicle may be obtained via a gravity sensor installed on the bottom of the underpan of the balance vehicle. The weight of the human body may not change greatly in a short period, therefore, whether the current user is the predetermined user may be determined via the weight of the human body, such that the balance vehicle may be prevented from being stolen and the property loss of the user may be avoided.

The first physiological characteristics information may include the weight, and may also include area occupied by a foot.

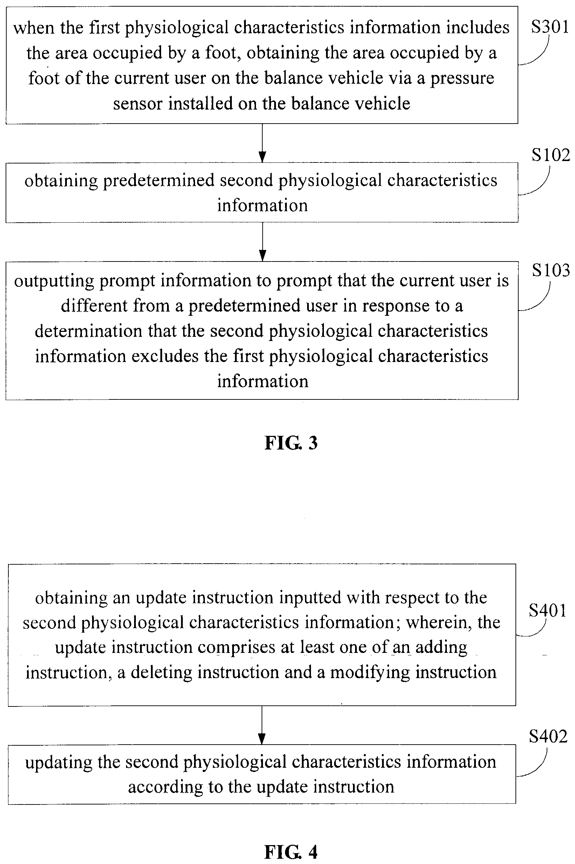

Fig. 3 is a flow diagram illustrating another anti-theft method for a balance vehicle according to an exemplary embodiment. As shown in Fig. 3, when the first physiological characteristics information includes the area occupied by a foot, the above step S101 may include step S301.

In step S301, the area occupied by a foot of the current user on the balance vehicle may be obtained via a pressure sensor installed on the balance vehicle.

In the embodiment, the area occupied by a foot of the current user on the balance vehicle may be obtained via a gravity sensor installed on the bottom of the underpan of the balance vehicle. The size of a foot of the human body may not change and may be recognizable, therefore, whether the current user is the predetermined user may be determined via the area occupied by a foot of the human body, such that the accuracy of the determination may be guaranteed, the balance vehicle may be prevented from being stolen, and the property loss of the user may be avoided.

Of course, in order to improve the accuracy of the determination, whether the current user is the predetermined user may be determined via the weight and the area occupied by a foot, such that prompt information may be outputted when it is determined that the current user is different from the predetermined user.

In one embodiment, the outputting prompt information may include controlling the balance vehicle to output the prompt information, wherein, the prompt information may be outputted by at least one of displaying content and playing voice.

In the embodiment, the prompt information may be outputted directly via the balance vehicle, such as the balance vehicle may generate an audio alarm, so that the attention of the people around the balance vehicle may be caught, which may stop the thief from stealing the balance vehicle and avoid property loss of the user.

In another embodiment, the outputting prompt information may also include controlling a terminal associated with the balance vehicle to output the prompt information, wherein, the prompt information is outputted by at least one of displaying content and playing voice.

In the embodiment, the terminal associated with the balance vehicle may be controlled to output the prompt information, such as the owner's terminal being bound to the balance vehicle may be controlled to output the prompt information so as to prompt the owner that the balance vehicle is stolen. For example, the prompt information may be outputted by displaying content such as a short message, and may be outputted by playing voice, the user may be informed easily.

Of course, in order to achieve better results, the balance vehicle and the terminal associated with the balance vehicle may be controlled to output the prompt information concurrently.

Fig. 4 is a flow diagram illustrating another anti-theft method for a balance vehicle according to an exemplary embodiment. As shown in Fig. 4, in one embodiment, the above method may include steps S401 to S402.

In step S401, an update instruction inputted with respect to the second physiological characteristics information may be obtained, wherein, the update instruction may include at least one of an adding instruction, a deleting instruction and a modifying instruction.

In step S402, the second physiological characteristics information may be updated according to the update instruction.

In the embodiment, when the user wants to perform adding, deleting and modifying operations to the predetermined second physiological characteristics information, the update instruction with respect to the second physiological characteristics information may be inputted, such as an adding instruction, a deleting instruction and a modifying instruction, so that the second physiological characteristics information may be updated.

For example, the owner of the balance vehicle may create an account and set a login password. The predetermined second physiological characteristics information may be stored in the account. The owner may input the correct login password to login the account, and then may update the second physiological characteristics information. In this way, other people may not update the second physiological characteristics information, the security of the balance vehicle may be guaranteed.

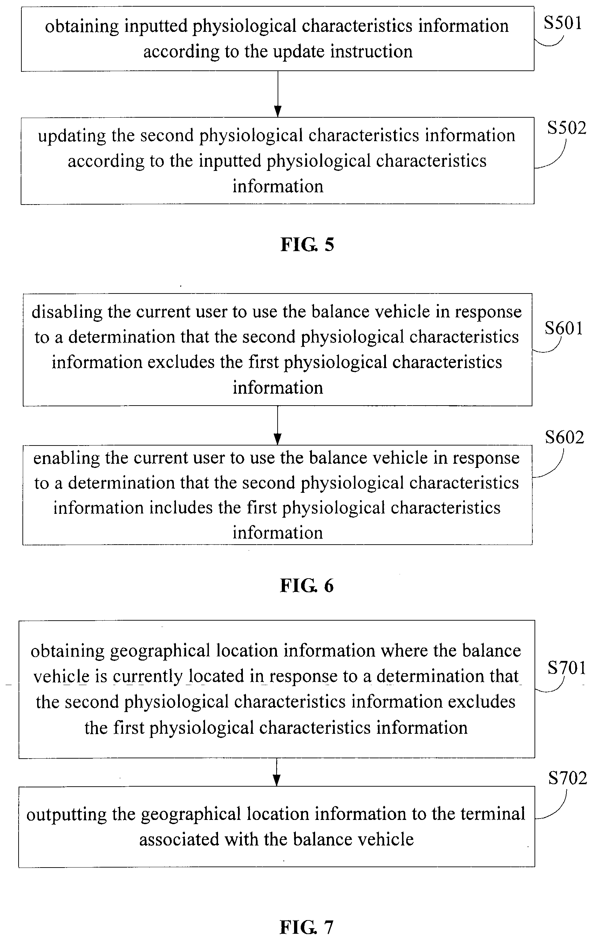

Fig. 5 is a flow diagram illustrating step S402 of another anti-theft method for a balance vehicle according to an exemplary embodiment. As shown in Fig. 5, in one embodiment, when the update instruction includes at least one of the adding instruction and the modifying instruction, the above step S402 may include steps S501to S502.

In step S501, inputted physiological characteristics information may be obtained according to the update instruction.

In step S502, the second physiological characteristics information may be updated according to the inputted physiological characteristics information.

In the embodiment, when the update instruction is received, the physiological characteristics information inputted by the user may be obtained. The predetermined second physiological characteristics information may be updated according to the physiological characteristics information. Such that the realtime and accuracy of the second physiological characteristics information may be guaranteed, and the accuracy of the determination that whether the balance vehicle is stolen may be guaranteed.

When obtaining inputted physiological characteristics information, the physiological characteristics information may be obtained via any one of the followings.

In method one, the physiological characteristics information inputted via a terminal may be obtained.

In the embodiment, the user may directly input the physiological characteristics information on the terminal, such as 53kg of the weight, 370cm² of the area occupied by feet. In this way, the user may input the physiological characteristics information on the terminal, which is very convenience.

Method two, the physiological characteristics information of a user on the balance vehicle may be obtained.

In the embodiment, the physiological characteristics information of the user may be obtained via the gravity sensor and the pressure sensor installed on the balance vehicle, such as the weight and the area occupied by a foot of the human body. In this way, the accuracy of the second physiological characteristics information may be guaranteed, and the accuracy of the determination that whether the balance vehicle is stolen may be guaranteed.

Fig. 6 is a flow diagram illustrating another anti-theft method for a balance vehicle according to an exemplary embodiment. As shown in Fig. 6, in one embodiment, the above method may include steps S601 to S602.

In step S601, the current user may be disabled to use the balance vehicle in response to a determination that the second physiological characteristics information excludes the first physiological characteristics information.

In step S602, the current user may be enabled to use the balance vehicle in response to a determination that the second physiological characteristics information includes the first physiological characteristics information.

In the embodiment, in order to prevent the balance vehicle from being stolen, when it is determined that the second physiological characteristics information excludes the first physiological characteristics information (namely when the current user is different from the predetermined user), the current user may be disabled to use the balance vehicle, such as the balance vehicle may be locked automatically. When the second physiological characteristics information includes the first physiological characteristics information, the current user may be enabled to use the balance vehicle.

Fig. 7 is a flow diagram illustrating another anti-theft method for a balance vehicle according to an exemplary embodiment. As shown in Fig. 7, in one embodiment, the above method may include steps S701 to S702.

In step S701, geographical location information where the balance vehicle is currently located may be obtained in response to a determination that the second physiological characteristics information excludes the first physiological characteristics information.

In step S702, the geographical location information may be outputted to the terminal associated with the balance vehicle.

In the embodiment, when it is determined that the second physiological characteristics information excludes the first physiological characteristics information (namely when the current user is different from the predetermined user), the geographical location information of the balance vehicle may be obtained, and the geographical location information may be transmitted to the terminal associated with the balance vehicle. For example, the geographical location information may be transmitted to the terminal of the owner. In this way, while the balance vehicle is stolen, the owner may find the balance vehicle according to the geographical location information, and the property loss of the user may be avoided.

The following is the device in the embodiments of the present disclosure which may be used to perform the method in the embodiments of the present disclosure.

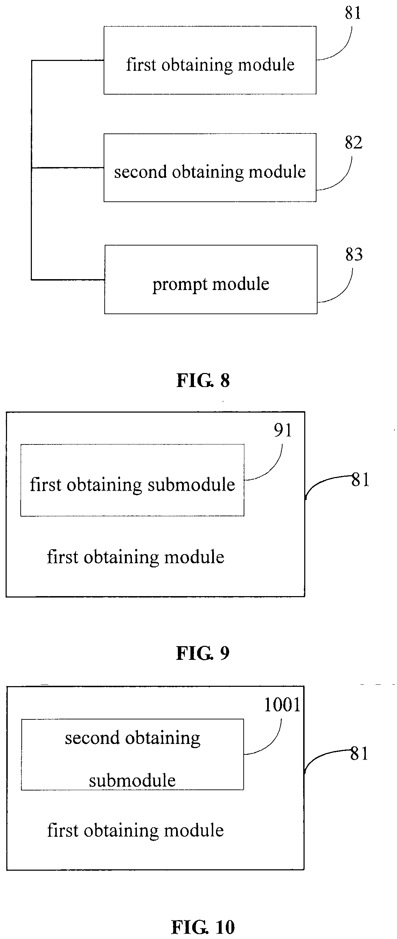

Fig. 8 is a block diagram illustrating an anti-theft device for a balance vehicle according to an exemplary embodiment. The device may be implemented as the balance vehicle or the terminal device controlling the balance vehicle using software, hardware, or the combination thereof. As shown in Fig. 8, the anti-theft device for a balance vehicle may include: a first obtaining module 81 configured to obtain first physiological characteristics information of a current user on the balance vehicle; a second obtaining module 82 configured to obtain predetermined second physiological characteristics information; a prompt module configured 83 to output prompt information to prompt that the current user is different from a predetermined user in response to a determination that the second physiological characteristics information obtained by the second obtaining module excludes the first physiological characteristics information obtained by the first obtaining module.

In the embodiment, the first obtaining module may obtain the first physiological characteristics information of the current user on the balance vehicle (such as weight and area occupied by a foot of the current user), the second obtaining module may obtain predetermined second physiological characteristics information (such as physiological characteristics information of the owner of the balance vehicle), the prompt module may output prompt information to prompt the user that the current user is different from a predetermined user when the second physiological characteristics information excludes the first physiological characteristics information (namely the physiological characteristics information of the current user is not the predetermined physiological characteristics information). Such that the balance vehicle may be prevented from being stolen and the property loss of the user may be avoided.

The second physiological characteristics information may be one or more, and may belong to one user or multiple users. When determining whether the second physiological characteristics information includes the first physiological characteristics information or not, it is expected to determine whether the first physiological characteristics information is the same as or similar to the second physiological characteristics information. Whether it is similar means whether the value of the first physiological characteristics is in an acceptable error range of the value of the second physiological characteristics, when it is in the error range, it may be determined that the second physiological characteristics information includes the first physiological characteristics information.

In one embodiment, the first physiological characteristics information may include at least one of weight and area occupied by a foot.

Fig. 9 is a block diagram illustrating a first obtaining module of an anti-theft device for a balance vehicle according to an exemplary embodiment.

As shown in Fig. 9, in one embodiment, the first physiological characteristics information may include the weight, the first obtaining module 81 may include a first obtaining submodule 91 configured to obtain the weight of the current user on the balance vehicle via a gravity sensor installed on the balance vehicle.

In the embodiment, the weight of the current user on the balance vehicle may be obtained via a gravity sensor installed on the bottom of the underpan of the balance vehicle. The weight of the human body may not change greatly in a short period, therefore, whether the current user is the predetermined user may be determined via the weight of the human body, such that the balance vehicle may be prevented from being stolen and the property loss of the user may be avoided.

Fig. 10 is a block diagram illustrating a first obtaining module of another anti-theft device for a balance vehicle according to an exemplary embodiment.

As shown in Fig. 10, in one embodiment, the first physiological characteristics information may include the area occupied by a foot, the first obtaining module 81 may include a second obtaining submodule 1001 configured to obtain the area occupied by a foot of the current user on the balance vehicle via a pressure sensor installed on the balance vehicle.

In the embodiment, the area occupied by a foot of the current user on the balance vehicle may be obtained via a gravity sensor installed on the bottom of the underpan of the balance vehicle. The size of a foot of the human body may not change and may be recognizable, therefore, whether the current user is the predetermined user may be determined via the area occupied by a foot of the human body, such that the accuracy of the determination may be guaranteed, the balance vehicle may be prevented from being stolen, and the property loss of the user may be avoided.

Of course, in order to improve the accuracy of the determination, whether the current user is the predetermined user may be determined via the weight and the area occupied by a foot, such that prompt information may be outputted when it is determined that the current user is different from the predetermined user.

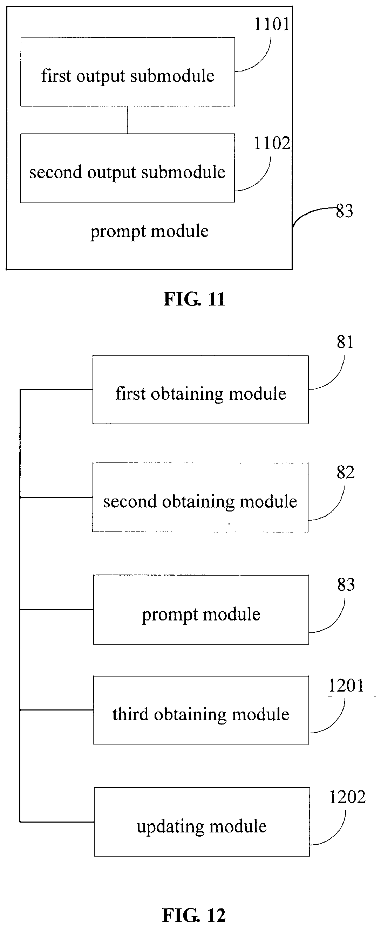

Fig. 11 is a block diagram illustrating a prompt module of another anti-theft device for a balance vehicle according to an exemplary embodiment.

As shown in Fig. 11, in one embodiment, the prompt module may include at least one of a first output submodule 1101 and a second output submodule 1102.

The first output submodule 1101 may be configured to control the balance vehicle to output the prompt information.

In the embodiment, the prompt information may be outputted directly via the balance vehicle, such as the balance vehicle may generate an audio alarm, so that the attention of the people around the balance vehicle may be caught, which may stop the thief from stealing the balance vehicle and avoid property loss of the user.

The second output submodule 1102 may be configured to control a terminal associated with the balance vehicle to output the prompt information, wherein, the prompt information is outputted by at least one of displaying content and playing voice.

In the embodiment, the terminal associated with the balance vehicle may be controlled to output the prompt information, such as the owner's terminal being bound to the balance vehicle may be controlled to output the prompt information so as to prompt the owner that the balance vehicle is stolen. For example, the prompt information may be outputted by displaying content such as a short message, and may be outputted by playing voice, the user may be informed easily.

Of course, in order to achieve better results, the balance vehicle and the terminal associated with the balance vehicle may be controlled to output the prompt information concurrently.

Fig. 12 is a block diagram illustrating another anti-theft device for a balance vehicle according to an exemplary embodiment.

As shown in Fig. 12, in one embodiment, the above device may also include a third obtaining module 1201 configured to obtain an update instruction inputted with respect to the second physiological characteristics information; an updating module 1202 configured to update the second physiological characteristics information according to the update instruction obtained by the third obtaining module; wherein, the update instruction comprises at least one of an adding instruction, a deleting instruction and a modifying instruction.

In the embodiment, when the user wants to perform adding, deleting and modifying operations to the predetermined second physiological characteristics information, the update instruction with respect to the second physiological characteristics information may be inputted, such as an adding instruction, a deleting instruction and a modifying instruction, so that the second physiological characteristics information may be updated.

For example, the owner of the balance vehicle may create an account and set a login password. The predetermined second physiological characteristics information may be stored in the account. The owner may input the correct login password to login the account, and then may update the second physiological characteristics information. In this way, other people may not update the second physiological characteristics information, the security of the balance vehicle may be guaranteed.

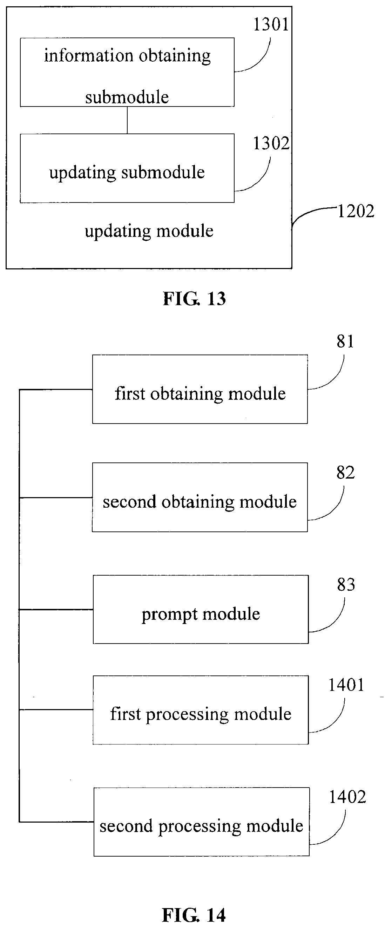

Fig. 13 is a block diagram illustrating an updating module of another anti-theft device for a balance vehicle according to an exemplary embodiment.

As shown in Fig. 13, in one embodiment, when the update instruction comprises at least one of the adding instruction and the modifying instruction, the updating module 1201 may include an information obtaining submodule 1301 configured to obtain inputted physiological characteristics information according to the update instruction; an updating submodule 1302 configured to update the second physiological characteristics information according to the inputted physiological characteristics information obtained by the information obtaining submodule.

In the embodiment, when the update instruction is received, the physiological characteristics information inputted by the user may be obtained. The predetermined second physiological characteristics information may be updated according to the physiological characteristics information. Such that the realtime and accuracy of the second physiological characteristics information may be guaranteed, and the accuracy of the determination that whether the balance vehicle is stolen may be guaranteed.

When obtaining inputted physiological characteristics information, the physiological characteristics information may be obtained via any one of the followings.

In method one, the physiological characteristics information inputted via a terminal may be obtained.

In the embodiment, the user may directly input the physiological characteristics information on the terminal, such as 53kg of the weight, 370cm² of the area occupied by feet. In this way, the user may input the physiological characteristics information on the terminal, which is very convenience.

In method two, the physiological characteristics information of a user on the balance vehicle may be obtained.

In the embodiment, the physiological characteristics information of the user may be obtained via the gravity sensor and the pressure sensor installed on the balance vehicle, such as the weight and the area occupied by a foot of the human body. In this way, the accuracy of the second physiological characteristics information may be guaranteed, and the accuracy of the determination that whether the balance vehicle is stolen may be guaranteed.

Fig. 14 is a block diagram illustrating another anti-theft device for a balance vehicle according to an exemplary embodiment.

As shown in Fig. 14, in one embodiment, the above device may also include a first processing module 1401 configured to disable the current user to use the balance vehicle in response to a determination that the second physiological characteristics information excludes the first physiological characteristics information; a second processing module 1402 configured to enable the current user to use the balance vehicle in response to a determination that the second physiological characteristics information includes the first physiological characteristics information.

In the embodiment, in order to prevent the balance vehicle from being stolen, when it is determined that the second physiological characteristics information excludes the first physiological characteristics information (namely when the current user is different from the predetermined user), the current user may be disabled to use the balance vehicle, such as the balance vehicle may be locked automatically. When the second physiological characteristics information includes the first physiological characteristics information, the current user may be enabled to use the balance vehicle.

According to a third aspect of the embodiments of the present disclosure, an anti-theft device for a balance vehicle is provided, comprising: a processor; a memory for storing processor-executable instructions; wherein the processor is configured to: obtain first physiological characteristics information of a current user on the balance vehicle; obtain predetermined second physiological characteristics information; output prompt information to prompt that the current user is different form a predetermined user in response to a determination that the second physiological characteristics information excludes the first physiological characteristics information.

The above processor may further be configured such that the first physiological characteristics information comprises at least one of weight and area occupied by a foot.

The above processor may further be configured such that the first physiological characteristics information comprises the weight; the obtain first physiological characteristics information of a current user on the balance vehicle comprises: obtain the weight of the current user on the balance vehicle via a gravity sensor installed on the balance vehicle.

The above processor may further be configured such that the first physiological characteristics information comprises the area occupied by a foot; the obtain first physiological characteristics information of a current user on the balance vehicle comprises: obtain the area occupied by a foot of the current user on the balance vehicle via a pressure sensor installed on the balance vehicle.

The above processor may further be configured such that the output prompt information comprises at least one of: control the balance vehicle to output the prompt information; control a terminal associated with the balance vehicle to output the prompt information; wherein, the prompt information is outputted by at least one of displaying content and playing voice.

The above processor may further be configured to obtain an update instruction inputted with respect to the second physiological characteristics information; update the second physiological characteristics information according to the update instruction; wherein, the update instruction comprises at least one of an adding instruction, a deleting instruction and a modifying instruction.

The above processor may further be configured such that when the update instruction comprises at least one of the adding instruction and the modifying instruction, the update the second physiological characteristics information according to the update instruction comprises: obtain inputted physiological characteristics information according to the update instruction; update the second physiological characteristics information according to the inputted physiological characteristics information.

The above processor may further be configured such that the obtain inputted physiological characteristics information comprises: obtain the physiological characteristics information inputted via a terminal; or obtain the physiological characteristics information of a user on the balance vehicle.

The above processor may further be configured to disable the current user to use the balance vehicle in response to a determination that the second physiological characteristics information excludes the first physiological characteristics information; enable the current user to use the balance vehicle in response to a determination that the second physiological characteristics information includes the first physiological characteristics information.

The above processor may further be configured to obtain geographical location information where the balance vehicle is currently located in response to a determination that the second physiological characteristics information excludes the first physiological characteristics information; output the geographical location information to the terminal associated with the balance vehicle.

The above processor may further be configured to obtain geographical location information where the balance vehicle is currently located in response to a determination that the second physiological characteristics information excludes the first physiological characteristics information; output the geographical location information to the terminal associated with the balance vehicle.

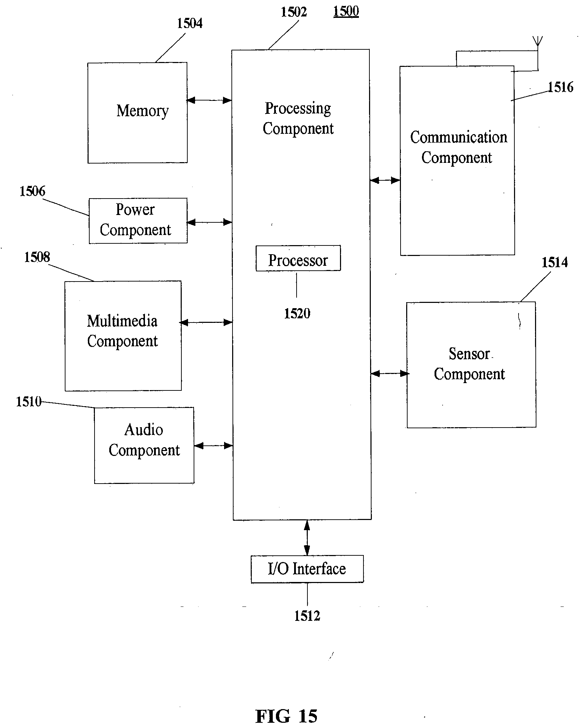

Fig. 15 is a block diagram illustrating an anti-theft device for a balance vehicle according to an exemplary embodiment. For example, the device 1500 may be a mobile phone, a computer, a digital broadcast terminal, a messaging device, a gaming console, a tablet, a medical device, an exercise equipment, a personal digital assistant, and the like.

The device 1500 may include one or more of the following components: a processing component 1502, a memory 1504, a power component 1506, a multimedia component 1508, an audio component 1510, an input/output (I/O) interface 1512, a sensor component 1514, and a communication component 1516.

The processing component 1502 typically controls overall operations of the device 1500, such as the operations associated with display, data communications, and recording operations. The processing component 1502 may include one or more processors 1520 to execute instructions to perform all or part of the steps in the above described methods. Moreover, the processing component 1502 may include one or more modules which facilitate the interaction between the processing component 1502 and other components. For instance, the processing component 1502 may include a multimedia module to facilitate the interaction between the multimedia component 1508 and the processing component 1502.

The memory 1504 is configured to store various types of data to support the operation of the device 1500. Examples of such data include instructions for any applications or methods operated on the device 1500, environmental states, times, etc. The memory 1504 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 1506 provides power to various components of the device 1500. The power component 1506 may include a power management system, one or more power sources, and any other components associated with the generation, management, and distribution of power in the device 1500.

The multimedia component 1508 includes a screen providing an output interface between the device 1500 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action.

The audio component 1510 is configured to output and/or input audio signals. For example, the audio component 1510 includes a microphone ("MIC") configured to receive an external audio signal when the device 1500 is in an operation mode, such as a recording mode and a voice recognition mode. The received audio signal may be further stored in the memory 1504 or transmitted via the communication component 1516. In some embodiments, the audio component 1510 further includes a speaker to output audio signals.

The I/O interface 1512 provides an interface between the processing component 1502 and peripheral interface modules, such as a keyboard, a click wheel, buttons, and the like. The buttons may include, but are not limited to, a home button, a volume button, a starting button, and a locking button.

The sensor component 1514 includes one or more sensors to provide status assessments of various aspects of the device 1500. For instance, the sensor component 1514 may detect an open/closed status of the device 1500, relative positioning of components, e.g., the display and the keypad, of the device 1500, a change in position of the device 1500 or a component of the device 1500, a presence or absence of user contact with the device 1500, an orientation or an acceleration/deceleration of the device 1500, and a change in temperature of the device 1500. The sensor component 1514 may include a proximity sensor configured to detect the presence of nearby objects without any physical contact. The sensor component 1514 may also include a light sensor, such as a CMOS or CCD image sensor, for use in imaging applications. In some embodiments, the sensor component 1514 may also include an accelerometer sensor, a gyroscope sensor, a magnetic sensor, a pressure sensor, or a temperature sensor.

The communication component 1516 is configured to facilitate communication, wired or wirelessly, between the device 1500 and other devices. The device 1500 can access a wireless network based on a communication standard, such as WiFi, 2G, or 3G, or a combination thereof. In one example embodiment, the communication component 1516 receives a broadcast signal or broadcast associated information from an external broadcast management system via a broadcast channel. In one example embodiment, the communication component 1516 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In example embodiments, the device 1500 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In example embodiments, there is also provided a non-transitory computer readable storage medium including instructions, such as included in the memory 1504, executable by the processor 1520 in the device 1500, for performing the above-described methods. For example, the non-transitory computer-readable storage medium may be a ROM, a random access memory (RAM), a CD-ROM, a magnetic tape, a floppy disc, an optical data storage device, and the like.

A non-transitory computer readable storage medium having stored therein instructions that, when executed by the processor of the device 1500, causes the device 1500 to perform the above described anti-theft method for a balance vehicle, comprising: obtaining first physiological characteristics information of a current user on the balance vehicle; obtaining predetermined second physiological characteristics information; outputting prompt information to prompt that the current user is different from a predetermined user in response to a determination that the second physiological characteristics information excludes the first physiological characteristics information.

In one embodiment, the first physiological characteristics information comprises at least one of weight and area occupied by a foot.

In one embodiment, the first physiological characteristics information comprises the weight; the obtaining first physiological characteristics information of a current user on the balance vehicle comprises: obtaining the weight of the current user on the balance vehicle via a gravity sensor installed on the balance vehicle.

In one embodiment, the first physiological characteristics information comprises the area occupied by a foot; the obtaining first physiological characteristics information of a current user on the balance vehicle comprises: obtaining the area occupied by a foot of the current user on the balance vehicle via a pressure sensor installed on the balance vehicle.

In one embodiment, the outputting prompt information comprises at least one of: controlling the balance vehicle to output the prompt information; controlling a terminal associated with the balance vehicle to output the prompt information; wherein, the prompt information is outputted by at least one of displaying content and playing voice.

In one embodiment, the method further comprising: obtaining an update instruction inputted with respect to the second physiological characteristics information; updating the second physiological characteristics information according to the update instruction; wherein, the update instruction comprises at least one of an adding instruction, a deleting instruction and a modifying instruction.

In one embodiment, when the update instruction comprises at least one of the adding instruction and the modifying instruction, the updating the second physiological characteristics information according to the update instruction comprises: obtaining inputted physiological characteristics information according to the update instruction; updating the second physiological characteristics information according to the inputted physiological characteristics information.

In one embodiment, the obtaining inputted physiological characteristics information comprises: obtaining the physiological characteristics information inputted via a terminal; or obtaining the physiological characteristics information of a user on the balance vehicle.

In one embodiment, the method further comprising: disabling the current user to use the balance vehicle in response to a determination that the second physiological characteristics information excludes the first physiological characteristics information; enabling the current user to use the balance vehicle in response to a determination that the second physiological characteristics information includes the first physiological characteristics information.

In one embodiment, the method further comprising: obtaining geographical location information where the balance vehicle is currently located in response to a determination that the second physiological characteristics information excludes the first physiological characteristics information; outputting the geographical location information to the terminal associated with the balance vehicle.

In one embodiment, the method further comprising: obtaining geographical location information where the balance vehicle is currently located in response to a determination that the second physiological characteristics information excludes the first physiological characteristics information; outputting the geographical location information to the terminal associated with the balance vehicle.

Other embodiments of the invention will be apparent to those skilled in the art from consideration of the specification and practice of the disclosures herein. This application is intended to cover any variations, uses, or adaptations of the disclosure following the general principles thereof and including such departures from the present disclosure as come within known or customary practice in the art. It is intended that the specification and embodiments be considered as illustrative only, with a true scope of the disclosure being indicated by the following claims.

It will be appreciated that the present disclosure is not limited to the exact construction that has been described above and illustrated in the accompanying drawings, and that various modifications and changes can be made without departing from the scope thereof. It is intended that the scope of the invention only be limited by the appended claims.

## Claims

1. An anti-theft method for a balance vehicle, comprising:
obtaining (S101) first physiological characteristics information of a current user on the balance vehicle;
obtaining (S102) predetermined second physiological characteristics information;
outputting (S103) prompt information to prompt that the current user is different from a predetermined user in response to a determination that the second physiological characteristics information excludes the first physiological characteristics information,
**characterized in that** the first physiological characteristics information comprises the area occupied by a foot, and wherein the obtaining (S101) first physiological characteristics information of a current user on the balance vehicle comprises: obtaining (S301) the area occupied by a foot of the current user on the balance vehicle via a pressure sensor installed on the balance vehicle.

2. The method of claim 1, wherein the first physiological characteristics information further comprises weight.

3. The method of claim 2, wherein;
the obtaining (S101) first physiological characteristics information of a current user on the balance vehicle further comprises:
obtaining (S201) the weight of the current user on the balance vehicle via a gravity sensor installed on the balance vehicle.

4. The method of claim 1, wherein the outputting prompt information comprises at least one of:
controlling the balance vehicle to output the prompt information;
controlling a terminal associated with the balance vehicle to output the prompt information;
wherein, the prompt information is outputted by at least one of displaying content and playing voice.

5. The method of claim 1, further comprising:
obtaining (S401) an update instruction inputted with respect to the second physiological characteristics information;
updating (S402) the second physiological characteristics information according to the update instruction;
wherein, the update instruction comprises at least one of an adding instruction, a deleting instruction and a modifying instruction.

6. The method of claim 5, when the update instruction comprises at least one of the adding instruction and the modifying instruction, the updating the second physiological characteristics information according to the update instruction comprises:
obtaining (S501) inputted physiological characteristics information according to the update instruction;
updating (S502) the second physiological characteristics information according to the inputted physiological characteristics information.

7. The method of claim 6, wherein the obtaining inputted physiological characteristics information comprises:
obtaining the physiological characteristics information inputted via a terminal; or
obtaining the physiological characteristics information of a user on the balance vehicle.

8. The method of any one of claims 1 to 7, further comprising:
disabling (S601) the current user to use the balance vehicle in response to a determination that the second physiological characteristics information excludes the first physiological characteristics information;
enabling (S602) the current user to use the balance vehicle in response to a determination that the second physiological characteristics information includes the first physiological characteristics information.

9. An anti-theft device for a balance vehicle comprising:
a first obtaining module (81) configured to obtain first physiological characteristics information of a current user on the balance vehicle;
a second obtaining module (82) configured to obtain predetermined second physiological characteristics information;
a prompt module (83) configured to output prompt information to prompt that the current user is different from a predetermined user in response to a determination that the second physiological characteristics information obtained by the second obtaining module (82) excludes the first physiological characteristics information obtained by the first obtaining module (81),
**characterized in that** the first physiological characteristics information comprises the area occupied by a foot, and wherein the first obtaining module (81) comprises: a second obtaining submodule (1001) configured to obtain the area occupied by a foot of the current user on the balance vehicle via a pressure sensor installed on the balance vehicle.

10. The device of claim 9, wherein the first physiological characteristics information further comprises weight.

11. The device of claim 10, wherein
the first obtaining module (81) further comprises:
a first obtaining submodule (91) configured to obtain the weight of the current user on the balance vehicle via a gravity sensor installed on the balance vehicle.

12. The device of claim 9, wherein the prompt module (83) comprises at least one of:
a first output submodule (1101) configured to control the balance vehicle to output the prompt information;
a second output submodule (1102) configured to control a terminal associated with the balance vehicle to output the prompt information;
wherein, the prompt information is outputted by at least one of displaying content and playing voice.

13. The device of claim 9, further comprising:
a third obtaining module (1201) configured to obtain an update instruction inputted with respect to the second physiological characteristics information;
an updating module (1202) configured to update the second physiological characteristics information according to the update instruction obtained by the third obtaining module (1201);
wherein, the update instruction comprises at least one of an adding instruction, a deleting instruction and a modifying instruction.

## Patentansprüche

1. Diebstahlsicherungsverfahren für ein Gleichgewichtsfahrzeug, das umfasst:
Erhalten (S101) von ersten Informationen über physiologische Charakteristiken eines aktuellen Benutzers auf dem Gleichgewichtsfahrzeug;
Erhalten (S102) von vorbestimmten zweiten Informationen über physiologische Charakteristiken;
Ausgeben (S103) von Meldeinformationen, die melden, dass sich der aktuelle Benutzer von einem vorbestimmten Benutzer unterscheidet, in Reaktion auf eine Bestimmung, dass die zweiten Informationen über physiologische Charakteristiken die ersten Informationen über physiologische Charakteristiken ausschließen,
**dadurch gekennzeichnet, dass**
die ersten Informationen über physiologische Charakteristiken den Bereich umfassen, der von einem Fuß eingenommen wird, und wobei das Erhalten (S101) von ersten Informationen über physiologische Charakteristiken eines aktuellen Benutzers auf dem Gleichgewichtsfahrzeug umfasst: Erhalten (S301) des Bereichs, der von einem Fuß des aktuellen Benutzers auf dem Gleichgewichtsfahrzeug eingenommen wird, über einen Drucksensor, der an dem Gleichgewichtsfahrzeug installiert ist.

2. Verfahren nach Anspruch 1, bei dem die ersten Informationen über physiologische Charakteristiken ferner ein Gewicht umfassen.

3. Verfahren nach Anspruch 2, bei dem:
das Erhalten (S101) von ersten Informationen über physiologische Charakteristiken eines aktuellen Benutzers auf dem Gleichgewichtsfahrzeug ferner umfasst:
Erhalten (S201) des Gewichts des aktuellen Benutzers auf dem Gleichgewichtsfahrzeug über einen Gravitationssensor, der an dem Gleichgewichtsfahrzeug installiert ist.

4. Verfahren nach Anspruch 1, bei dem das Ausgeben von Meldeinformationen mindestens eines umfasst von:
Steuern des Gleichgewichtsfahrzeugs zum Ausgeben der Meldeinformationen;
Steuern eines Bildschirms, der dem Gleichgewichtsfahrzeug zugeordnet ist, zum Ausgeben der Meldeinformationen;
wobei die Meldeinformationen durch mindestens eines von Anzeigen von Inhalt und Abspielen einer Stimme ausgegeben werden.

5. Verfahren nach Anspruch 1, das ferner umfasst:
Erhalten (S401) einer Aktualisierungsanweisung, die bezüglich der zweiten Informationen über physiologische Charakteristiken eingegeben wird;
Aktualisieren (S402) der zweiten Informationen über physiologische Charakteristiken entsprechend der Aktualisierungsanweisung;
wobei die Aktualisierungsanweisung mindestens eine einer Hinzufügeanweisung, einer Löschanweisung und einer Modifizieranweisung umfasst.

6. Verfahren nach Anspruch 5, wobei dann, wenn die Aktualisierungsanweisung mindestens eine der Hinzufügeanweisung und der Modifizieranweisung umfasst, das Aktualisieren der zweiten Informationen über physiologische Charakteristiken entsprechend der Aktualisierungsanweisung umfasst:
Erhalten (S501) von eingegebenen Informationen über physiologische Charakteristiken entsprechend der Aktualisierungsanweisung;
Aktualisieren (S502) der zweiten Informationen über physiologische Charakteristiken entsprechend den eingegebenen Informationen über physiologische Charakteristiken.

7. Verfahren nach Anspruch 6, bei dem das Erhalten von eingegebenen Informationen über physiologische Charakteristiken umfasst:
Erhalten der Informationen über physiologische Charakteristiken über einen Bildschirm; und
Erhalten der Informationen über physiologische Charakteristiken eines Benutzers auf dem Gleichgewichtsfahrzeug.

8. Verfahren nach einem der Ansprüche 1 bis 7, das ferner umfasst:
Unterbinden (S601) der Benutzung des Gleichgewichtsfahrzeugs durch den aktuellen Benutzer in Reaktion auf eine Bestimmung, dass die zweiten Informationen über physiologische Charakteristiken die ersten Informationen über physiologische Charakteristiken ausschließen;
Erlauben (S602) der Benutzung des Gleichgewichtsfahrzeugs durch den aktuellen Benutzer in Reaktion auf eine Bestimmung, dass die zweiten Informationen über physiologische Charakteristiken die ersten Informationen über physiologische Charakteristiken einschließen.

9. Diebstahlsicherungsvorrichtung für ein Gleichgewichtsfahrzeug, die umfasst:
ein erstes Erhaltungsmodul (81), das derart ausgeführt ist, dass es erste Informationen über physiologische Charakteristiken eines aktuellen Benutzers auf dem Gleichgewichtsfahrzeug erhält;
ein zweites Erhaltungsmodul (82), das derart ausgeführt ist, dass es vorbestimmte zweite Informationen über physiologische Charakteristiken erhält;
ein Meldemodul (83), das derart ausgeführt ist, dass es Meldeinformationen ausgibt, die melden, dass sich der aktuelle Benutzer von einem vorbestimmten Benutzer unterscheidet, in Reaktion auf eine Bestimmung, dass die zweiten Informationen über physiologische Charakteristiken, die von dem zweiten Erhaltungsmodul (82) erhalten werden, die ersten Informationen über physiologische Charakteristiken, die von dem ersten Erhaltungsmodul (81) erhalten werden, ausschließen,
**dadurch gekennzeichnet, dass**
die ersten Informationen über physiologische Charakteristiken den Bereich umfassen, der von einem Fuß eingenommen wird, und wobei das erste Erhaltungsmodul (81) umfasst: ein zweites Erhaltungssubmodul (1001), das derart ausgelegt ist, dass es den Bereich, der von einem Fuß des aktuellen Benutzers auf dem Gleichgewichtsfahrzeug eingenommen wird, über einen Drucksensor, der an dem Gleichgewichtsfahrzeug installiert ist, erhält.

10. Vorrichtung nach Anspruch 9, bei der die ersten Informationen über physiologische Charakteristiken ferner ein Gewicht umfassen.

11. Vorrichtung nach Anspruch 10, bei der
das erste Erhaltungsmodul (81) ferner umfasst:
ein erstes Erhaltungssubmodul (91), das derart ausgeführt ist, dass es das Gewicht des aktuellen Benutzers auf dem Gleichgewichtsfahrzeug über einen Gravitationssensor, der an dem Gleichgewichtsfahrzeug installiert ist, erhält.

12. Vorrichtung nach Anspruch 9, bei der das Meldemodul (83) mindestens eines umfasst von:
einem ersten Ausgabesubmodul (1101), das derart ausgeführt ist, dass es das Gleichgewichtsfahrzeug zum Ausgeben der Meldeinformationen steuert;
ein zweites Ausgabesubmodul (1102), das derart ausgeführt ist, dass es einen Bildschirm, der dem Gleichgewichtsfahrzeug zugeordnet ist, zum Ausgeben der Meldeinformationen steuert;
wobei die Meldeinformationen durch mindestens eines von Anzeigen von Inhalt und Abspielen einer Stimme ausgegeben werden.

13. Vorrichtung nach Anspruch 9, die ferner umfasst:
ein drittes Erhaltungsmodul (1201), das derart ausgeführt ist, dass es eine Aktualisierungsanweisung, die bezüglich der zweiten Informationen über physiologische Charakteristiken eingegeben werden, erhält;
ein Aktualisierungsmodul (1202), das derart ausgeführt ist, dass es die zweiten Informationen über physiologische Charakteristiken entsprechend der Aktualisierungsanweisung, die von dem dritten Erhaltungsmodul (1201) erhalten wird, aktualisiert;
wobei die Aktualisierungsanweisung mindestens eine einer Hinzufügeanweisung, einer Löschanweisung und einer Modifizieranweisung umfasst.

## Revendications

1. Procédé antivol pour un véhicule à auto-équilibrage, comprenant le fait de:
obtenir (S101) des informations sur les premières caractéristiques physiologiques d'un utilisateur actuel du véhicule à auto-équilibrage;
obtenir (S102) des informations sur les deuxièmes caractéristiques physiologiques prédéterminées;
sortir (S103) des informations d'interrogation pour demander si l'utilisateur actuel est différent d'un utilisateur prédéterminé en réponse à une détermination que les informations sur les deuxièmes caractéristiques physiologiques excluent les informations sur les premières caractéristiques physiologiques,
**caractérisé par le fait que** les premières informations sur les caractéristiques physiologiques comprennent la superficie occupée par un pied, et où l'obtention (S101) des informations sur les premières caractéristiques physiologiques d'un utilisateur actuel du véhicule à auto-équilibrage comprend le fait de: obtenir (S301) la superficie occupée par un pied de l'utilisateur actuel du véhicule à auto-équilibrage par l'intermédiaire d'un capteur de pression installé sur le véhicule à auto-équilibrage.

2. Procédé selon la revendication 1, dans lequel les informations sur les premières caractéristiques physiologiques comprennent par ailleurs un poids.

3. Procédé selon la revendication 2, dans lequel:
l'obtention (S101) des informations sur les premières caractéristiques physiologiques d'un utilisateur actuel du véhicule à auto-équilibrage comprend par ailleurs le fait de:
obtenir (S201) le poids de l'utilisateur actuel du véhicule à auto-équilibrage par l'intermédiaire d'un capteur de gravité installé sur le véhicule à auto-équilibrage.

4. Procédé selon la revendication 1, dans lequel la sortie des informations d'interrogation comprend au moins l'un parmi le fait de:
commander le véhicule à auto-équilibrage pour qu'il sorte les informations d'interrogation;
commander un terminal associé au véhicule à auto-équilibrage pour qu'il sorte les informations d'interrogation;
dans lequel les informations d'interrogation sont sorties par au moins l'un parmi l'affichage du contenu et la reproduction vocale.

5. Procédé selon la revendication 1, comprenant par ailleurs le fait de:
obtenir (S401) une instruction de mise à jour entrée en ce qui concerne les informations sur les deuxièmes caractéristiques physiologiques;
mettre à jour (S402) les informations sur les deuxièmes caractéristiques physiologiques selon l'instruction de mise à jour;
dans lequel l'instruction de mise à jour comprend au moins l'une parmi une instruction d'addition, une instruction de suppression et une instruction de modification.

6. Procédé selon la revendication 5, dans lequel, lorsque l'instruction de mise à jour comprend au moins l'une parmi l'instruction d'addition et l'instruction de modification, la mise à jour des informations sur les deuxièmes caractéristiques physiologiques selon l'instruction de mise à jour comprend le fait de:
obtenir (S501) les informations sur les caractéristiques physiologiques entrées selon l'instruction de mise à jour;
mettre à jour (S502) les informations sur les deuxièmes caractéristiques physiologiques selon les informations sur les caractéristiques physiologiques entrées.

7. Procédé selon la revendication 6, dans lequel l'obtention des informations sur les caractéristiques physiologiques entrées comprend le fait de:
obtenir les informations sur les caractéristiques physiologiques entrées par l'intermédiaire d'un terminal; ou
obtenir les informations sur les caractéristiques physiologiques d'un utilisateur du véhicule à auto-équilibrage.

8. Procédé selon l'une quelconque des revendications 1 à 7, comprenant par ailleurs le fait de:
empêcher (S601) l'utilisateur actuel d'utiliser le véhicule à auto-équilibrage en réponse à une détermination que les informations sur les deuxièmes caractéristiques physiologiques excluent les informations sur les premières caractéristiques physiologiques;
permettre (S602) à l'utilisateur actuel d'utiliser le véhicule à auto-équilibrage en réponse à une détermination que les informations sur les deuxièmes caractéristiques physiologiques incluent les informations sur les premières caractéristiques physiologiques.

9. Dispositif antivol pour un véhicule à auto-équilibrage comprenant:
un premier module d'obtention (81) configuré pour obtenir les informations sur les premières caractéristiques physiologiques d'un utilisateur actuel du véhicule à auto-équilibrage;
un deuxième module d'obtention (82) configuré pour obtenir les informations sur les deuxièmes caractéristiques physiologiques prédéterminées;
un module d'interrogation (83) configuré pour sortir les informations d'interrogation pour demander si l'utilisateur actuel est différent d'un utilisateur prédéterminé en réponse à une détermination que les informations sur les deuxièmes caractéristiques physiologiques obtenues par le deuxième module d'obtention (82) excluent les informations sur les premières caractéristiques physiologiques obtenues par le premier module d'obtention (81),
**caractérisé par le fait que** les informations sur les premières caractéristiques physiologiques comprennent la superficie occupée par un pied, et où le premier module d'obtention (81) comprend: un deuxième sous-module d'obtention (1001) configuré pour obtenir la superficie occupée par un pied de l'utilisateur actuel du véhicule à auto-équilibrage par l'intermédiaire d'un capteur de pression installé sur le véhicule à auto-équilibrage.

10. Dispositif selon la revendication 9, dans lequel les informations sur les premières caractéristiques physiologiques comprennent par ailleurs un poids.

11. Dispositif selon la revendication 10, dans lequel
le premier module d'obtention (81) comprend par ailleurs:
un premier sous-module d'obtention (91) configuré pour obtenir le poids de l'utilisateur actuel du véhicule à auto-équilibrage par l'intermédiaire d'un capteur de gravité installé sur le véhicule à auto-équilibrage.

12. Dispositif selon la revendication 9, dans lequel le module d'interrogation (83) comprend au moins l'un parmi:
un premier sous-module de sortie (1101) configuré pour commander le véhicule à auto-équilibrage pour qu'il sorte les informations d'interrogation;
un deuxième sous-module de sortie (1102) configuré pour commander un terminal associé au véhicule à auto-équilibrage pour qu'il sorte les informations d'interrogation;
dans lequel les informations d'interrogation sont sorties par au moins l'un parmi l'affichage du contenu et la reproduction vocale.

13. Dispositif selon la revendication 9, comprenant par ailleurs:
un troisième module d'obtention (1201) configuré pour obtenir une instruction de mise à jour entrée en ce qui concerne les informations sur les deuxièmes caractéristiques physiologiques;
un module de mise à jour (1202) configuré pour mettre à jour les informations sur les deuxièmes caractéristiques physiologiques selon l'instruction de mise à jour obtenue par le troisième module d'obtention (1201);
dans lequel l'instruction de mise à jour comprend au moins l'une parmi une instruction d'addition, une instruction de suppression et une instruction de modification.
